# EUROPEAN PATENT SPECIFICATION

(11) **EP 1 108 743 B1**
(45) Date of publication and mention of the grant of the patent: **15.11.2006**
(21) Application number: 00126977.8
(22) Date of filing: 08.12.2000
(51) Int. Cl.: C08K 3/00, C08K 3/22, C12N 7/00, C12N 7/02, B03C 1/01

(54) **Separation of viruses and detection of viruses**
Trennung von Viren und Nachweis von Viren
Séparation de virus et détection de virus

(30) Priority: 08.12.1999 JP 34904199; 16.02.2000 JP 2000038010
(43) Date of publication of application: 20.06.2001
(73) Proprietor: JSR Corporation, Tokyo 104-0045 (JP)
(72) Inventor: Sato, Kouei, Sashima-gun, Ibaraki-ken (JP); Tanaka, Takeshi, Tokyo (JP); Murata, Mitsuhiro, Ushiku-shi, Ibaraki-ken (JP); Nishida, Shozou, Iizuka-shi, Fukuoka-ken (JP); Hikata, Mikio, Tsuchiura-shi, Ibaraki-ken (JP); Kasai, Kiyoshi, Kameyama-shi, Mie-ken (JP)
(74) Representative: TBK-Patent

(56) References cited:
- EP-A- 0 709 680
- WO-A-00/42432
- WO-A-97/00896
- WO-A-97/11160
- WO-A-99/35500
- DE-A- 19 800 294
- FR-A- 2 463 807
- PATENT ABSTRACTS OF JAPAN vol. 2000, no. 14, 5 March 2001 (2001-03-05) & JP 2000 306718 A (JSR CORP), 2 November 2000 (2000-11-02)

## Description

### BACKGROUND OF THE INVENTION

### 1. Field of the Invention

This invention relates to a method of separating viruses and.a method of detecting viruses using the same.

### 2. Description of the Prior Art

Viruses may cause various diseases of human beings, animals and vegetables. For detecting viruses to diagnose diseases, it is very important to ascertain causative viruses. As conventional methods for detecting viruses to make diagnosis, it is common to assay virus antigens or antiviral antibodies immunologically. Since, however, viruses or antiviral antibodies are in a small quantity for several weeks to several months after viral infection, the viruses can not be detected by such immunological assay in some cases. The period for which they can not be detected is called a window period (blank period). If some patients standing in such a period donate blood, the blood thus donated may often be well infectious, and has a possibility of bringing many unspecified blood-transfused patients or blood preparation utilizer patients into harmful condition. Accordingly, in order to make the window period as short as possible, it is a pressing need to develop a technique by which any viruses standing below the limit of immunological detection can be detected at a high sensitivity.

In recent years, nucleic-acid amplification techniques as typified by the polymerase chain reaction process (hereinafter "PCR process") has brought about a possibility for enabling detection of viruses present even in a trace quantity. However, the PCR process and so forth also require special equipment and high-grade techniques for such detection of viruses present in a trace quantity, and it is very difficult to put it into practice with ordinary equipment and in a simple way. Accordingly, in order to solve such problems, methods of separating viruses present in a specimen are used.

As a typical example of conventional methods for separating viruses, ultracentrifugation is available, which, however, requires an expensive machinery and a long time and also can not make simultaneous treatment of a large number of specimens with ease, and is hard to regard as a simple method. Also reported is chromatography making use of a heparin-Sepharose carrier, utilizing the characteristic that a type B hepatitis virus surface antigen (HBs antigen) combines with heparin. It is also difficult for this method to make simultaneous treatment of a large number of specimens. As the other methods, also available are methods of causing viruses to sediment by a method making use of ammonium sulfate or combination of polyethylene glycol or polyanion with a divalent ion, e.g., a particulate substance having acidic groups (Japanese Post-examination Publication (Kokoku) No. 6-22627) or combination of particles with a divalent metal (Japanese Laid-open Publication (Kokai) No. 6-217767), or by a method in which a water soluble polymeric substance having cationic groups is added to remove viruses (Japanese Laid-open Publication (Kokai) No. 4-342536). These methods, however, involve a difficulty that samples must be purified after viruses have been separated by sedimentation, because of problems such that reagents to be mixed and proteins mixed in a large quantity in the viruses thus separated cause the inhibition of PCR.

WO-A-99/35500 discloses magnetic and heat-sensitive particles having a particle size between 0.05 and 10 µm used for diagnostic tests for determining the presence of infectious genes and to extract DNA or RNA or proteins.

WO-A-97/00896 discloses mono-dispersed magnetisable microsphere latex particles consisting of a hydrophobic polymer matrix and magnetisable fillers. Said latexes may be used in biology, in particular for diagnostic and affinity chromatography methods to bind two biological molecules.

EP-A-0,709,680 discloses magnetic particles used in an immunoassay method using gelatine-coated magnetic particles having a particle size of 1.0 to 10 µm. Such particles may be used to bind antibodies, hormones, viral antigens, DNA, RNA of viruses.

FR-A-2,463,807 discloses a method for fixing of biological molecules on magnetic supports comprising contacting a solution containing a biological molecule with a magnetic support consisting of magnetic particles of vinyl aromatic polymers so as to adsorb the biological molecules on the support and separating the biological support from the solution.

DE-A-19800294 discloses magnetic particles consisting of a magnetic core and a polymeric matrix in which the magnetic core is completely encapsulated so that chemically coupling of biological molecules is possible.

### SUMMARY OF THE INVENTION

Accordingly, an object of the present invention is to solve the above problems in virus separation methods to provide virus-binding particles which enable simultaneous and simple treatment of a large number of specimens by a simple means and which are readily adaptable to automation and do not adversely affect any nucleic-acid amplification tests, and to provide a virus-separating reagent, a virus separation method and a virus detection method which make use of such particles.

As a result of researches, as a means for solving the above problems, the present inventors have accomplished development of the following methods involving virus-binding particles.

The present invention provides a method of separating viruses, comprising the steps of:
adding virus-binding particles as defined in claim 1 to a sample which possiby contains viruses, to allow the viruses to bind to the particles; and
separating from the sample the particles to which the viruses have bound.

The present invention further provides a method of detecting viruses, comprising the steps of:
adding virus-binding particles as defined in claim 4 to a sample which possiby contains viruses, to allow the viruses to bind to the particles;
separating viruses from the sample by separating from the sample the particles to which the viruses have bound, to collect the viruses; and
subjecting the viruses thus separated, to a nucleic-acid amplification test.

### DETAILED DESCRIPTION OF THE INVENTION

The present invention will be described below in detail.

### - Virus-binding particles -

In the present invention, virus-binding particles refer to particles which adsorb viruses present in blood or humors and from which the viruses having been adsorbed thereon are to be separated. The viruses having been separated by the particles are used to make nucleic-acid extraction, examination and diagnosis, in particular, examination and diagnosis involving nucleic-acid amplification. The virus-binding particles of the present invention have at least one of a cationic group and an anionic group (both inclusive of the state of salts) at particle surfaces, and have a particle diameter of from 0.05 to 300 µm.

The virus-binding particles used in the present invention may be any of water-insoluble materials without any particular limitations, and are comprised of particles formed of such materials, and at least one of a cationic group and an anionic group which is present at the surfaces of the particles.

The cationic group in the present invention may include an amino group, such as a primary amino group, a secondary amino group and a tertiary amino group, a quaternary ammonium group, an imino group such as a primary imino group, a secondary imino group and a tertiary imino group, a quaternary iminium group, an amidino group such as a primary amidino group, a secondary amidino group and a tertiary amidino group, a quaternary amidinium group, a hydradino group such as a primary hydradino group, a secondary hydradino group and a tertiary hydradino group, a quaternary hydrazinium group, or an anionic nitrogen-containing cyclic group such as pyridyl groups and a quaternary pyridinium group.

In the present invention, the cationic group includes a group capable of combining with a proton such as an amino group to form a cation, and a group formed by the reaction of such a group with an acid to produce a salt in which a cationic moiety is formed by the group.

In the present invention, the cationic group may inhibit nucleic-acid amplification such as PCR if it dissolves out in water, buffer solutions, blood or humors, and hence must be chemically combined with the particles. It may be present in an amount of at least 1 x 10⁻¹⁰ mol, typically from 1 x 10⁻¹⁰ to 1 x 10⁻² mol, preferably from 1 x 10⁻⁹ to 1 x 10⁻³ mol, and more preferably from 1 x 10⁻⁸ to 1 x 10⁻³ mol, per g of particles on the average. If the cationic group is present in an amount less than 1 x 10⁻¹⁰ mol, the particles may have an insufficient virus-separating ability. Though the above upper limit is not critical, it is often difficult to introduce the cationic group in an amount more than 1 x 10⁻² mol.

The particles having a cationic group can be produced by, e. g. , (1) a process in which monomer components containing a cationic monomer are polymerized; (2) a process in which a monomer is polymerized in the presence of a radical polymerization initiator having a cationic group; and (3) a process in which a compound having a cationic group is made to combine with particles.

### (1) Process in which monomer components containing a cationic monomer are polymerized:

In this process, usable cationic monomers may include, for example,
aminoalkyl group-containing (meth)acrylates ["(meth)acryl..." is meant to be acryl... or methacryl... or a mixture of these; the same applies hereinafter] such as 2-dimethylaminoethyl (meth)acrylate, 2-diethylaminoethyl (meth)acrylate. 2-dimethylaminopropyl (meth)acrylate and 3-dimethylaminopropyl (meth)acrylate, and quaternary salts of these with methylene chloride, dimethyl sulfate, diethyl sulfate or the like;
aminoalkoxyalkyl-group-containing (meth)acrylates such as 2-(dimethylaminoethoxy)ethyl (meth)acrylate, 2-(diethylaminoethoxy)ethyl (meth)acrylate and 3-(dimethylaminoethoxy)propyl (meth)acrylate, and quaternary salts of these with methylene chloride, dimethyl sulfate, diethyl sulfate or the like;
N-aminoalkyl-group-containing (meth)acrylamides such as N-(2-dimethylaminoethyl) (meth)acrylamide, N-(2-diethylaminoethyl) (meth)acrylamide, N-(2-dimethylaminopropyl) (meth)acrylamide and N-(3-dimethylaminopropyl) (methl)acrylamide, and quaternary salts of these with methylene chloride, dimethyl sulfate, diethyl sulfate or the like. In particular, 2-dimethylaminoethyl (meth)acrylate, N-(2-diethylaminoethyl) (meth)acrylamide, and quaternary salts of these with methylene chloride are preferred.

Any of these may be used singly or in combination of two or more.

The monomer copolymerizable with the cationic monomer may include crosslinkable monomers and non-crosslinkable and nonionic monomers as shown below.

The crosslinkable monomers may include, for example, divinyl monomers, trivinyl monomers and tetravinyl monomers, such as divinylbenzene, divinylbiphenyl, ethylene glycol di(meth)acrylate, diethylene glycol di(meth)acrylate, triethylene glycol di(meth)acrylate, tetraethylene glycol di(meth)acrylate, propylene glycol di(meth)acrylate, dipropylene glycol di(meth)acrylate, tripropylene glycol di(meth)acrylate, tetrapropylene glycol di(meth)acrylate, 1,4-butanediol di(meth)acrylate, 1,6-hexanediol di(meth)acrylate, neopentyl glycol di(meth)acrylate, 2,2'-bis[4-(meth)acryloyloxypropioxyphenyl]propane, 2,2'-bis[4-(meth)acryloyloxydiethoxydiphenyl]propane, glycerol tri(meth)acrylate, trimethylolpropane tri(meth)acrylate, and pentaerythritol tetra(meth)acrylate. In particular, divinylbenzene, ethylene glycol dimethacrylate and trimethylolpropane trimethacrylate are preferred.

Any of these may be used singly or in combination of two or more.

The copolymerizable non-crosslinkable and nonionic monomers are monomers which are copolymerizable with either the cationic monomer or the crosslinkable monomers or both and are non-crosslinkable and nonionic.

Such monomers may include aromatic vinyl monomers such as styrene. α-methylstyrene, p-methylstyrene and halogenated styrenes; unsaturated nitriles such as acrylonitrile; acrylates and methacrylates, such as methyl acrylate, methyl methacrylate, ethyl acrylate, ethyl methacrylate, butyl acrylate, butyl methacrylate, cyclohexyl methacrylate. 2-ethylhexyl acrylate, 2-ethylhexyl methacrylate, lauryl acrylate, lauryl methacrylate, glycidyl acrylate, glycidyl methacrylate, 2-hydroxyethyl acrylate and 2-hydroxyethyl methacrylate; diolefines such as butadiene and isoprene; vinyl carboxylate esters such as vinyl acetate; and vinyl or vinylidene chlorides such as vinyl chloride and vinylidene chloride. In particular, styrene, α-methylstyrene, acrylonitrile, methyl methacrylate, butyl methacrylate, 2-hydroxyethyl acrylate and cyclohexyl methacrylate are preferred.

Any of these monomers may be used singly or in combination of two or more.

Polymeric components containing the cationic monomer described above can be obtained by emulsion polymerization, suspension polymerization or the like in the presence of a polymerization initiator in an aqueous dispersion medium.

The polymerization initiator is exemplified by redox type water-soluble polymerization initiators such as persulfates, hydrogen peroxide-ferrous chloride, and cumene hydroperoxide-sodium ascorbate; and oil-soluble polymerization initiators such as benzoyl peroxide, lauroyl peroxide, t-butyl peroxy-2-ethylhexanoate, and azobisisobutyronitrile.

Surface-active agents, dispersion stabilizers and so forth may also optionally be used.

### (2) Process making use of a radical polymerization initiator having a cationic group:

This radical polymerization initiator is one in the presence of which a polymer obtained by radical polymerization comes to have at its terminal a cationic group derived from the radical polymerization initiator.

Preferred radical polymerization initiators having a cationic group may include azobis type initiators having an amidino group, an imidino group or a pyridium group. Those having a 10-hour half-life temperature in the range of from 40 to 95°C are preferred because the polymerization can be carried out under mild conditions.

As preferred specific examples of such radical polymerization initiators having a cationic group, they may include the following:
2,2'-azobis(2-methyl-N-phenylpropionamidine) dihydrochloride (available as VA-545, trade name, from Wako Pure Chemical Industries, Ltd.):
2,2'-azobis[N-(4-chlorophenyl)-2-methylpropionamidine] dihydrochloride (available as VA-546, trade name, from Wako Pure Chemical Industries, Ltd.);
2,2'-azobis[N-(4-hydroxyphenyl)-2-methylpropionamidine] dihydrochloride (available as VA-548, trade name, from Wako Pure Chemical Industries, Ltd.);
2,2'-azobis[2-methyl-N-(phenylmethyl)-propionamidine] dihydrochloride (available as VA-552, trade name, from Wako Pure Chemical Industries, Ltd.):
2,2'-azobis[2-methyl-N-(2-propenyl)propionamidine] dihydrochloride (available as VA-553, trade name, from Wako Pure Chemical Industries, Ltd.):
2,2'-azobis(2-methylpropionamidine) dihydrochloride (available as V-50, trade name, from Wako Pure Chemical Industries. Ltd.);
2,2'-azobis[N-(2-hydroxyethyl)-2-methylpropionamidine] dihydrochloride (available as VA-558, trade name, from Wako Pure Chemical Industries, Ltd.);
2,2'-azobis[N-(2-carboxyethyl)-2-methylpropionamidine] hydrate (available as VA-057, trade name, from Wako Pure Chemical Industries, Ltd.);
2,2'-azobis[2-methyl-(5-methyl-2-imidazolin-2-yl)propane] dihydrochloride (available as VA-041, trade name, from Wako Pure Chemical Industries, Ltd.);
2,2'-azobis[2-(2-imidazolin-2-yl)propane] dihydrochloride (available as VA-044, trade name, from Wako Pure Chemical Industries, Ltd.);
2,2'-azobis[2-(4.5,6,7-tetrahydro-1H-1,3-diazepin-2-yl)propa ne] dihydrochloride (available as VA-054, trade name, from Wako Pure Chemical Industries, Ltd.);
2,2'-azobis[2-(3,4,5,6-tetrahydropyrimidin-2-yl)propane] dihydrochloride (available as VA-058, trade name, from Wako Pure Chemical Industries, Ltd.);
2,2'-azobis[2-(5-hydroxy-3,4,5,6-tetrahydropyrimidin-2-yl)pr opane] dihydrochloride (available as VA-059, trade name, from Wako Pure Chemical Industries, Ltd.);
2,2'-azobis{2-[1-(2-hydroxyethyl)-2-imidazaolin-2-yl)propane} dihydrochloride (available as VA-060, trade name, from Wako Pure Chemical Industries, Ltd.); and
2,2'-azobis[2-(2-imidazaolin-2-yl)propane] dihydrochloride (available as VA-061, trade name, from Wako Pure Chemical Industries, Ltd.).

In particular, use of 2,2'-azobis(2-methylpropionamidine) dihydrochloride (V-50).
2,2'-azobis(N-(2-carboxyethyl)-2-methylpropionamidine) hydrate (VA-057) or 2,2'-azobis[2-(2-imidazolin-2-yl)propane] dihydrochloride (VA-044) is preferred.

The radical polymerization initiator having a cationic group may preferably be used in an amount of from 0.1 to 10 parts by weight, and more preferably from 0.5 to 5 parts by weight, based on 100 parts by weight of the total sum of monomers used in the polymerization. Its use in an amount less than 0.1 parts by weight, which is too little, may make the resultant particles low cationic, and its use in an amount more than 10 parts by weight, which is in excess, may make the polymerization unstable, undesirably.

### (3) Process in which a compound having a cationic group is made to combine with particles:

A monomer having a functional group such as a carboxyl group, a hydroxyl group, an epoxy group, an amino group or an amide group is subjected to copolymerization or seed polymerization to produce a polymer having functional groups, and these functional groups are allowed to react as linking moieties, whereby the compound having a cationic group can be introduced to particle surfaces.

The monomer having a functional group for introducing the functional groups may include acrylic acid, methacrylic acid, crotonic acid, itaconic acid, fumaric acid, maleic acid, hydroxyethyl methacrylate, 2-hydroxyethyl acrylate, glycidyl methacrylate, acrylamide, methacrylamide, N-methylolmethacrylamide and N-isopropylacrylamide.

Cationic compounds to be reacted with functional groups introduced to particle surfaces as described above are: polyamine compounds classified into polyalkylimines such as polyvinylamine, polyallylamine, polyethyleneimine and polypropyleneimine.

The anionic group in the present invention may include a carboxyl group and a sulfonic acid group (-SO₃H). These anionic groups may be present in the state they have formed salts. In the present invention, the anionic group, made present in at least part of particles, and preferably at the surfaces of particles, may inhibit nucleic-acid amplification such as PCR if it dissolves out in water, buffer solutions, blood or humors, and hence must be chemically combined with the particles. It may be present in an amount of at least 1 x 10⁻¹⁰ mol, typically from 1 x 10⁻¹⁰ to 1 x 10⁻² mol, preferably from 1 x 10⁻⁹ to 1 x 10⁻³ mol, and more preferably from 1 x 10⁻⁸ to 1 x 10⁻³ mol, per 1 g of particles on the average. If the anionic group is present in an amount less than 1 x 10⁻¹⁰ mol, the particles may have an insufficient virus-separating ability. Though the above upper limit is not critical, it is often difficult to introduce the anionic group in an amount more than 1 x 10⁻² mol.

Such particles may include, e.g., surface-sulfonated particles, carboxyl group-containing particles, particles comprising synthetic polymeric particles with which a sulfonic acid group-containing monomer and/or a carboxyl group-containing monomer has or have been seed-polymerized or graft-polymerized, hydrogel particles comprised of a sulfonic acid group-containing monomer and a water-soluble crosslinkable monomer, polyanionic compound-fixed particles, and anionic inorganic particles. Without limitation to these, any particles may be used as long as they have an anionic group at at least part of the surface.

As the surface-sulfonated particles, usable are, e.g. , particles comprised of a polymer or copolymer of a polymeric compound having at least at the surface of a particle a functional group capable of being sulfonated as exemplified by a back-bone-chain or side-chain unsaturated double bond, an aromatic group, a primary or secondary amino group, a primary alkyl halide group, an aliphatic aldehyde, an aliphatic ketone, an aliphatic carboxylic acid, an aliphatic carboxylic anhydride residual group or a hydroxyl group present on the backbone or side chains and having been sulfonated at at least part of the surface to have a sulfonic acid group. As examples of the polymeric compound constituting the particles capable of being sulfonated at their surfaces, it may include addition polymerization type polymeric compounds such as polymers or copolymers of monomers capable of being sulfonated, as exemplified by styrene, α-methylstyrene, vinylnaphthalene, divinylbenzene, butadiene, isoprene and vinyl alcohol, and copolymers of any of these monomers with different polymerizable monomers; and condensation polymerization type polymeric compounds such as polycarbonates, polyesters, polyester ethers, polyaryl ethers, polyalkylene oxides, polysulfones, polyether sulfones, polyamides, polyimides, polyether imides, polyether ketones, polyurethanes, acetaldehyde condensation products of aromatic compounds, and polyethers.

The particles comprised of any of these polymeric compounds can be sulfonated by treating the particles with concentrated sulfuric acid, fuming sulfuric acid, sulfuric anhydride, a sulfuric anhydride-dioxane complex, a sulfuric anhydride-pyridine complex, chlorosulfonic acid or the like.

The carboxyl-containing particles may include polymer or copolymer particles of a carboxyl group-containing monomer. Here, the carboxyl group-containing monomer (hereinafter "carboxylic acid monomer") refers to a polymerizable monomer having in the molecule an addition-polymerizable unsaturated bond and a carboxyl group. As specific examples thereof, it may include acrylic acid, methacrylic acid, crotonic acid, itaconic acid, fumaric acid and maleic acid.

These polymer or copolymer particles can be synthesized by a conventional method of emulsion polymerization or suspension polymerization.

The particles comprising synthetic polymeric particles with which a sulfonic acid group-containing monomer (hereinafter "sulfonic acid monomer") and/or a carboxylic acid monomer has or have been seed-polymerized or graft-polymerized may include particles obtained by subjecting seed particles comprised of a synthetic macromolecule, to seed polymerization or copolymerization or graft polymerization or copolymerization together with a sulfonic acid monomer and/or a carboxylic acid monomer and further optionally with a different copolymerizable monomer other than these. Such particles can be synthesized by adding a monomer and a radical generator to seed particles dispersed in water containing a surface-active agent or in a water/polar solvent mixture, followed by reaction carried out at 50 to 100°C. Here, the seed particles comprised of a synthetic macromolecule may include polymer or copolymer particles of aromatic compounds containing a polymerizable double bond, such as styrene, α-methylstyrene, vinyltoluene and vinylnaphthalene; cyan compounds containing a polymerizable double bond, such as acrylonitrile, methacrylonitrile and vinylidene cyanide; polymerizable crosslinkable compounds such as divinylbenzene and ethylene glycol dimethacrylate; organic compounds containing a polymerizable double bond, such as vinyl chloride, vinylidene chloride, vinyl methyl ethyl ketone, vinyl methyl ether, vinyl acetate, vinyl formate, allyl acetate, methallyl acetate, acrylamide, methacrylamide, N-methylolmethaciylamide, N-isopropylacrylamide, glycidyl acrylate, glycidyl methacrylate, acrolein, methacrolein, allyl alcohol, 2-hydroxyethyl acrylate, 2-hydroxyethyl methacrylate, 2-methoxyethyl acrylate, n-butyl acrylate, sec-butyl acrylate, isobutyl acrylate, t-butyl acrylate, n-butyl methacrylate, sec-butyl methacrylate, isobutyl methacrylate, t-butyl methacrylate, methyl acrylate, ethyl acrylate, n-propyl acrylate, iso-propyl acrylate, methyl methacrylate, ethyl methacrylate, n-propyl methacrylate and iso-propyl methacrylate; and polymerizable cyclic compounds such as ethylene oxide, propylene oxide, 2-methyltetrahydroxyfuran, styrene oxide, butylene oxide and glycidyl ether.

The sulfonic acid monomer may include isoprenesulfonic acid, ethylenesulfonic acid, vinylsulfonic acid, styrenesulfonic acid, α-methylstyrenesulfonic acid, 2-acrylamido-2-methylpropanesulfonic acid, sulfoethyl acrylate and sulfonated dicyclopentadiene.

As the different polymerizable monomer, it may include aliphatic diene compounds such as 1,3-butadiene and isoprene; aromatic compounds containing a polymerizable double bond, such as styrene. α-methylstyrene and vinyltoluene; alkyl (metha)acrylates such as methyl acrylate, ethyl acrylate, 2-ethylhexyl acrylate, methyl methacrylate, ethyl methacrylate, 2-hydroxyethyl acrylate and 2-hydroxyethyl methacrylate; polymerizable cyan compounds such as acrylonitrile and methacrylonitrile; organic compounds containing a polymerizable double bond, such as vinyl chloride, vinylidene chloride, vinyl methyl ethyl ketone, vinyl methyl ether, vinyl acetate, vinyl formate, allyl acetate, methallyl acetate, acrylamide, methacrylamide, N-methylolmethacrylamide, N-isopropylacrylamide, glycidyl acrylate, glycidyl methacrylate, acrolein, methacrolein and allyl alcohol; and polymerizable cyclic compounds such as ethylene oxide, propylene oxide, 2-methyltetrahydroxyfuran, styrene oxide, butylene oxide and glycidyl ether.

As the hydrogel particles comprised of a sulfonic acid monomer and a water-soluble crosslinkable monomer, usable are, e.g., hydrogel particles comprised of a polymer of the sulfonic acid monomer described above with a water-soluble crosslinkable monomer such as N,N'-methylenebisacrylamide, Such particles can be obtained by vigorously mixing an aqueous solution of the sulfonic acid monomer, the water-soluble crosslinkable monomer and a radical reaction initiator with a non-polar solvent to which a surface-active agent has been added, to form an water-in-oil type reverse micelle, followed by reaction carried out at 50 to 100°C.

The polyanionic compound-fixed particles may include particles comprising particles having a functional group such as an epoxy group, an amino group, an aldehyde group, a carboxyl group, a hydroxyl group or an acid chloride group on particle surfaces of which a polyanion having in the molecule a plurality of sulfonic acid groups and/or carboxyl groups has been supported directly or via a coupling agent or a spacer. Here, the polyanion may include, e.g., polymeric compounds obtained by polymerizing or copolymerizing a sulfonic acid group-containing monomer and/or a carboxylic acid monomer and further optionally a monomer other than these; tungstophosphoic acids; and polyphosphoric acids.

As the anionic inorganic particles, SiO₂-Al₂O₃-based particles on which sulfonic acid groups have been introduced, such as activated clay or the like may preferably be used. Also, in the case of particulate materials of glass, silica, alumina, activated carbon and so forth, their surfaces may be coated with a polymer having a sulfonic acid group or a carboxyl group. Such a polymer may include polyacrylic acid, polymethacrylic acid, polystyrenesulfonic acid, and copolymers of a monomer which is a constituent unit of any of these polymers with a hydrophilic monomer. The hydrophilic monomer may include hydrophilic acrylate monomers and hydrophilic methacrylate monomers. Apart from this, in the case of glass or silica, the carboxyl group can be introduced by first introducing an amino group using a silane-coupling agent such as γ-aminopropyltriethdoxysilane, followed by reaction with succinic anhydride or with succinic acid in the presence of carbodimide. Also, the sulfonic acid group can be introduced by first introducing an aldehyde group by treatment with gultaldehyde often the treatment with the silane-coupling agent, followed by reaction thereof with 2-aminoethanesulfonic acid.

The virus-binding particles used in the present invention may comprise a magnetic material in the interiors, or at the surfaces, of the particles.

This magnetic material may preferably be incorporated only in the interiors and be not laid bare to the surfaces. In the present invention, the incorporation of a magnetic material in the virus-binding particles enables collection of particles by the action of magnetism possessed by the particles, and makes it unnecessary to collect them by centrifugation or the like. This not only makes it possible to shorten examination time, but also makes it easy to deal with the automation of examination and diagnosis. Such a magnetic material may include, e.g., ferrites of various types such as triion tetraoxide (Fe₃O₄) and γ-iron sesquioxide (γ-Fe₂O₃), or metals such as iron, manganese, cobalt and chromium or alloys of any of these metals, any of which may be used.

The magnetic material may be contained in an amount not less than 10% by weight, and particularly from 20 to 100% by weight, preferably 20 to 90% by weight, based on the total weight of the virus-binding particles. If it is in too small a quantity, no good magnetically separable properties may be attained in the virus-binding particles. As the result, in the method of separating viruses as described later, it takes fairly long time to separate the virus-binding particles from specimens such as blood and humors, and hence any high time efficiency is not obtainable in some cases, undesirably.

In order to incorporate the magnetic material in the virus-binding particles, the following methods may be employed.
(a) The magnetic material is dispersed in a polymerization component containing the cationic monomer or anionic monomer, and polymerization is carried out in that state. The polymerization may be carried out by a process such as conventional emulsion polymerization, suspension polymerization or dispersion polymerization.
(b) A polymerization component containing the cationic monomer or anionic monomer is polymerized to synthesize polymer particles, and thereafter magnetic material layers are formed on the particle surfaces.
(c) On the surfaces of the particles obtained in the above (a) or (b), polymer layers comprised of a polymerization component containing the cationic monomer or anionic monomer are further formed.

In some cases, the virus-binding particles thus obtained contain in their dispersion medium an emulsifier, a dispersant, unreacted monomers, a water-soluble polymer, a polymerization initiator decomposition product and so forth. These substances have a high possibility of turning to reaction inhibitors in the step of a nucleic-acid amplification test. Accordingly, they may preferably be removed from the dispersion medium of the virus-binding particles by. e.g., the method disclosed in Adv. Colloid Interface Sci., 81, 77-165 (1999).

### (Polyvalent Metal Compound)

In the present invention, a polyvalent metal compound may be added to a sample together with the virus-binding particles, whereby viruses can be made to bind to the virus-binding particles at a higher proportion in some cases.

Here, the polyvalent metal may include, e.g., Be, Mg, Sr, Ba, Ti, Zr, Cr, Mo, W, Mn, Fe, Ru, Os, Co, Rh, Ni, Pd, Pt, Cu, Ag, Au, Zn, Cd. Hg, Al, Ga, Si, Ge. Sn, Pb, P, As, Sb and Bi. The polyvalent metal compound refers to a compound capable of forming divalent or higher-valent cations upon dissociation in water, among chlorides, hydroxides, carbonic acid compounds, sulfuric acid compounds, nitric acid compounds, acetic acid compounds and chloric acid compounds of these polyvalent metals. Of these polyvalent metal compounds, magnesium chloride and the like are preferred.

The polyvalent metal compound may be used in such a quantity that it comes to be usually in a concentration of from 0.1 to 100 mmol/L in a reaction mixture formed when the virus-binding particles and a sample are mixed.

### - Virus-separating Reagent -

In the present invention, the virus-separating reagent comprises the above virus-binding particles and the above polyvalent metal compound.

The preparation method of the reagent is not limited. The virus-separating reagent may be in the form the polyvalent metal compound has been added in a dispersion prepared by dispersing the virus-binding particles in an aqueous medium, or in the form the dispersion and the polyvalent metal compound are stored separately so that they are mixed immediately before use.

Any viruses in a specimen such as plasma or serum are separated in a high efficiency, and hence, usually, the virus-binding particles are used not in column chromatography but in a batch process. Accordingly, the particles have particle diameters of from 0.05 µm to 300 µm, preferably from 0.1 µm to 100 µm, and more preferably from 0.2 µm to 80 µm. As long as the particles have particle diameters within this range, they are usable for what is intended in the present invention. Particles having particle diameters smaller than 0.05 µm are undesirable because such particles may make it necessary to carry out centrifugation at a larger number of revolutions or for a longer time of revolution when the virus-bound particles are separated from blood or a humor, so that the apparatus must be made larger in size or any high time efficiency can not be achieved. Particles having particle diameters larger than 300 µm are also undesirable because such particles may have a low efficiency for capturing viruses, and may make it unable in some cases to separate viruses well. Also, with regard to the particle shape of the virus-binding particles of the present invention, the particles need not be spherical, and may be irregular-shaped particles. As for the particle diameter of particles which are not spherical, an average value of the largest lengths and smallest breadths of individual particles is found.

### - Separation of Viruses -

The method of separating viruses according to the present invention comprises the steps of:
adding the above virus-binding particles to a sample which possiby contains viruses, to allow the viruses to bind to the particles; and
separating from the sample the particles to which the viruses have bound, to collect the viruses.

A method of separating viruses by means of the virus-separating reagent of the present invention will specifically be described below.

The virus-separating reagent used in the present invention has the ability to separate viruses of various types. For example, it can separate hepadnaviruses (such as hepatitis B virus), adenoviruses, flaviviruses (such as Japanese B encephalitis virus), herpesviruses (such as herpes simplex virus, varicella-zoster virus, cytomegalovirus, or EB virus), poxviruses, parvoviruses (such as adeno-related virus), orthomyxoviruses (such as influenza virus), rhabdoviruses (such as rabies virus), retroviruses (such as human immunodeficiency viruses) and hepatitis C virus.

Specimens for which the separation of viruses by the virus-separating reagent of the present invention is intended may include humors such as plasma, serum, cell lysate, urea and saliva, and cultured cell fragment fluid. Such specimens may be used as samples as they are, or may be used as samples after they have been diluted for some purposes.

The virus-binding particles used in the present invention are desirably added to a sample in the form of a virus-separating reagent prepared by the virus-binding particles in a medium such as saline. The quantity in which the virus-separating reagent used in the present invention is added in a sample depends on the concentration of viruses present in the sample. It may be so added that the virus-binding particles in the virus-separating reagent are usually in an amount of from 0.05 to 50% by weight, and preferably from 0.1 to 20% by weight, of the weight of the sample. Its addition in too small a quantity puts a limitation on the number of viruses which can bind to the virus-binding particles, resulting in a poor separation efficiency. Also, its addition in too large a quantity makes it necessary to use a detachment solution in a large quantity when bound viruses are detached in a post stage, resulting in a low separation efficiency.

Virus-binding particles which have adsorbed thereon the viruses in a sample are separated from the sample by centrifugation or natural sedimentation or, in the case when the virus-binding particles contain the magnetic material, by magnetic separation. Since the virus-binding particles used in the present invention have particle diameters in the range of from 0.05 to 300 µm, the particles can well be centrifugally separated by means of a centrifuge.

The virus-bound particles thus separated are optionally washed with a low-concentration buffer, and thereafter normally moved to the step of separating viruses from the particles. As the method of separating viruses from the particles, a method is available in which a salt solution is made to act to dissociate viruses from the virus-bound particles. As the salt solution, a high-concentration potassium bromide, sodium bromide, 1.5 M sodium chloride, 1 mM tungstophosphoric acid solution or 1 M sodium thiocyanate may be used. The viruses thus separated from the particles are further treated according to the separation purpose. For example, the viruses are subjected to extract on of nucleic acids. Here, the extraction of nucleic acid may be made as the viruses are kept bound to the particles. For example, a buffer may be added in a small quantity to the virus-binding particles separated from the sample, followed by heating to extract the nucleic acid of viruses directly, or a commercially available extraction reagent may directly be added to the virus-bound particles to extract the nucleic acid of viruses.

### - Detection of Viruses -

The method of detecting viruses according to the present invention comprises, after the step where the particles used in the present invention to which the viruses have bound as described above are separated and collected from the sample, the step of;
subjecting the viruses thus separated, to a nucleic-acid amplification test.

There are no particular limitations on the nucleic-acid amplification test (NAT) of viruses. For example, usable are the PCR (polymerization chain reaction) process of F. Hoffman-La Roche Ltd., the TMA (transcription mediated amplification-hybridization protection assay) process of Gen-Probe Inc., the LCR (ligase chain reaction) process of Abbott Labolatories, the ICAN (Isothermal and chimeric primer-initiated amplification of nucleic acid) process of Takara Shuzo Co., and the LAMP (Loop-mediated isothermal amplification of DNA) process of Eiken Chemical Co.

In the virus detection method utilizing this method of separating viruses, the viruses in a specimen to be subjected to the nucleic-acid amplification test have already been separated, and hence the viruses can efficiently be detected even when the viruses contained in the original specimen or in the sample are in a very small quantity.

The virus-binding particles used in the present invention can also be used to detect viral proteins in a sample.

Stated specifically, a method is available in which the virus-binding particles used in the present invention are mixed with a sample and the virus exosporium protein adsorbed on the particles is detected with a labeled antibody. In this method, the type of the labeled antibody may be changed to detect virus exosporium proteins of various types.

### EXAMPLES

Examples of the present invention are given below. The present invention should by no means be construed limitative to these Examples. In the following "part(s)" represents part(s) by weight unless otherwise noted.

The particle diameter of virus-binding particles obtained in these Examples and the quantity of cationic groups present in the particles were measured in the following way.

### - Measurement of particle diameter:

A photograph of particles was taken on an optical microscope, a scanning electron microscope or a transmission electron microscope, and particle diameters of 200 particles were measured to find their average value.

### - Quantity of cationic groups present in particles:

### (1) Conductivity titrimetry

The virus-binding particles were washed twice with pure water by centrifugation or magnetic separation, and a mixed-bed type ion-exchange resin was added in an amount of 5 g per 1 g of the particles. The mixture obtained was stirred for 1 hour, and thereafter the mixed-bed type ion-exchange resin was filtered off. This purification with the mixed-bed type ion-exchange resin was repeated twice. The resultant purified particles were titrated using a normal sulfuric acid solution as a titrant. This method was applied in Examples 1 to 3.

### (2) Non-aqueous titrimetry

The virus-binding particles were purified in the same manner as the above (1). Thereafter, the purified product was dried and was dispersed in chloroform, and then chloroform-insoluble matter was filtered off, and then the filtrate obtained was subjected to titration using a normal solution of perchloric acid/acetic acid solution to determine the quantity of cationic groups. This method was applied in Examples 4 to 6.

### - Quantity of anionic groups present in particles:

To about 10 g of the particles, 90 g of ion-exchanged water and 30 g of an anion/cation-exchange resin mixture (AMBERLITE MB3, trade name, available from Organo K.K.), and the mixture obtained was gently stirred for 1 hour. The ion-exchange resin was filtered off with a nylon mesh (48 meshes; pore size: 295 µm), and the quantity of the particles was determined, followed by conductivity titration using 0.01 mol/L of a sodium hydroxide solution to determine the quantity of anionic groups.

### Reference Example 1

### The Reference Examples are outside the scope of the invention

Acetone was added to an oily magnetic fluid MARPO MAGNA FV 55, trade name, available from Matsumoto Yushi Seiyaku, Co., Ltd. , to cause particles to undergo precipitation sedimentation, followed by drying to obtain a ferrite type superparamagnetic material having lipophilic-treated particle surfaces (particle diameter: 0.01 µm).

Then, to 40 parts (by weight; the same applies hereinafter) of the superparamagnetic material, 90 parts of cyclohexyl methacrylate, 10 parts of chloride of trimethylaminoethyl methacrylate and 3 parts of benzoyl peroxide (polymerization initiator) were added, and the resultant system was mixed and stirred to disperse the superparamagnetic material uniformly, thus a monomer composition was prepared.

Meanwhile, 10 parts of polyvinyl alcohol, 0.5 part of sodium laurate and 0.1 part of polyethylene oxide nonylphenyl ether were dissolved in 1,000 parts of water, thus an aqueous monomer composition was prepared.

In the aqueous monomer composition thus obtained, the above monomer composition in which the superparamagnetic material has been dispersed, was added, and the mixture was preliminarily stirred by means of a homogenizer, followed by dispersion treatment by means of an ultrasonic dispersion machine, thus a suspension (an oil-drop dispersion) was prepared in which oil drops (oil phase) of 1 µm in average particle diameter were dispersed in the aqueous medium.

Next, the suspension thus obtained was charged into a 2-liter volume three-necked flask having a stirrer, and this system was heated to a temperature of 75°C to polymerize (suspension polymerization) the monomers in oil drops over a period of 5 hours, in an atmosphere of nitrogen and with stirring, thus virus-binding particles of the present invention were produced.

### Reference Example 2

Virus-binding particles were produced in the same manner as in Reference Example 1 except that 10 parts of the chloride of trimethylaminoethyl methacrylate used therein was replaced with 10 parts of dimethylaminoethyl methacrylate.

### Reference Example 3

Virus-binding particles were produced in the same manner as in Reference Example 1 except that the cyclohexyl methacrylate used therein was used in an amount of 100 parts, the chloride of trimethylaminoethyl methacrylate was not used and 3 parts of the benzoyl peroxide (polymerization initiator) was replaced with 5 parts of 2,2'-azobis(2-amidinopropane) dibasic acid salt.

### Example 4

(1) Magnetic polymer particles were produced in the same manner as in Reference Example 1 except that the cyclohexyl methacrylate used therein was used in an amount of 95 parts and 10 parts of the chloride of trimethylaminoethyl methacrylate was replaced with 5 parts of methacrylic acid.
(2) The magnetic polymer particles obtained in the above (1) were dispersed in an aqueous 5 mM sodium hydroxide solution, followed by treatment at 80°C for 12 hours to obtain carboxy-modified magnetic polymer particles.
(3) 1 g of the carboxy-modified magnetic polymer particles thus obtained were added to 20 mL of a 10 mM HES buffer (pH 6), and 1 mL of an aqueous 30% polyethyleneimine (number-average molecular weight: 70.000) solution and 0.2 g of a water-soluble carbodiimide reagent, 1-ethyl-3-(3-dimethylaminopropyl)carbodiimide (EDC) hydrochloride were further added, where reaction was carried out at 20°C for 2 hours to obtain virus-binding particles of the present invention.
(4) To the reaction mixture obtained, 5 g of mixed-bed type ion-exchange resin was added, and thereafter the mixed-bed type ion-exchange resin was removed by filtration. This purification with the mixed-bed type ion-exchange resin was repeated twice, followed by drying.

The magnetic polymer particles thus dried were dissolved in 10 mL of chloroform, and the magnetic material was separated by filtration and was subjected to non-aqueous titration with 0.01 mol/L of a perchloric acid/acetic acid solution to find that the total amino nitrogen combined with the magnetic polymer particles obtained in the above (2) was in an amount of 45.0 µmol/g.

### Reference Example 5

(1) 1 g (dry weight) of the carboxy-modified magnetic polymer particles obtained in the same manner as in Example 4, step (2), were added to 20 mL of a 10 mM MES buffer, and 1 mL of an aqueous 1% poly-L-lysine hydrogen bromide (number-average molecular weight: 300,000) and 0.2 g of a water-soluble carbodiimide reagent, EDC hydrochloride (1-ethyl-3-(3-dimethylaminopropyl)carbodimide hydrochloride) were further added, where reaction was carried out at 20°C for 2 hours to obtain virus-binding particles, comprised of poly-L-lysine-fixed magnetic particles.
(2) The poly-L-lysine-fixed magnetic particles thus obtained were purified in the same manner as in Example 4, step (4), followed by drying. The resultant particles were dissolved in 10 mL of dimethyl sulfoxide, and the magnetic material was separated by filtration, which was then subjected to non-aqueous titration with 0.01 mol/L of a perchloric acid/acetic acid solution to find that the total amino nitrogen combined with the magnetic polymer particles obtained in the above (1) was in an amount of 22.0 µmol/g.

### Example 6

(1) Glycidyl-modified magnetic particles were obtained in the same manner as in Reference Example 1 except that 10 parts of the chloride of trimethylaminoethyl methacrylate was replaced with 10 parts of glycidyl methacrylate.
(2) 1 g (dry weight) of the glycidyl-modified magnetic particles obtained were suspended in 20 mL of distilled water containing 1% of pyridine, and 1 mL of an aqueous 30% polyethyleneimine (number-average molecular weight: 70,000) solution was further added, where reaction was carried out at 60°C for 24 hours to obtain virus-binding particles of the present invention, comprised of polyethyleneimine-fixed magnetic particles.
(3) The polyethyleneimine-fixed magnetic particles thus obtained were purified in the same manner as in Example 4, step (4), followed by drying. The resultant particles were dissolved in 10 mL of chloroform, and the magnetic material was separated by filtration, which was then subjected to non-aqueous titration with 0.01 mol/L of a perchloric acid/acetic acid solution to find that the total amino nitrogen combined with the magnetic particles obtained in the above (2) was in an amount of 20 µmol/g.

**Table 1**

| | Particle diameter (µm) | Cationic-group quantity (mol/g) |
|---|---|---|
| Example 1* | 1.2 | 5.4 x 10⁻⁷ |
| Example 2* | 3.7 | 7.8 X 10⁻⁸ |
| Example 3* | 1.1 | 5.0 X 10⁻⁷ |
| Example 4 | 0.7 | 4.5 X 10⁻⁶ |
| Example 5* | 0.8 | 1.1 X 10⁻⁶ |
| Example 6 | 1.2 | 1.0 X 10⁻⁵ |

| | | |
|---|---|---|
| * Reference Example | | |

### Example 7 (Test 1)

(1) Aqueous dispersions of the virus-binding particles obtained in Reference Examples 1 to 3 and 5 and Examples 4 and 6 were purified and thereafter the concentration of the particle solid-matter was adjusted to 5% using physiological saline solution (Experiment Nos. 1 to 6).
   An aqueous dispersion of the virus-binding particles obtained in Reference Example 2 was also purified and thereafter the concentration of the particle solid-matter was adjusted to 5% by weight using physiological saline solution, and then manganese dichloride was added so that the concentration thereof might become 100 mmol/L (Experiment No. 7).
(2) To 1 mL of human plasma containing 10⁴ copies/mL of HBV (hepatitis B virus). 100 µL of the particle suspension (5% by weight) obtained in the above (1) was added, followed by rotational stirring at room temperature for 10 minutes.

After the reaction was completed, the mixture was set on a magnetic separation stand to effect separation into particles and a supernatant, where the supernatant was discarded to obtain the particles. To the particles obtained in this way, 50 µL of an aqueous 1 M sodium thiocyanate was added, and the mixture obtained was stirred for 5 minutes, which was thereafter separated into particles and a supernatant on a magnetic separation stand to obtain the supernatant (separated fraction). Its final volume was about 50 µL.

The separated fraction supernatant was taken to effect nucleic-acid extraction by a conventional method, and the quantity of DNA was determined by the TaqMan PCR process, using ABI PRISM TM7700 Sequence Detection System (made by Perkin Elmer Applied Biosystems Corp.). To make the determination of DNA, in the course of amplification made 40 times, the number of cycles (Th cycles) where fluorescence intensity exceeded a stated reference value (threshold: Th) was extrapolated on a calibration curve of DNA quantity Th cycles which was prepared by making measurement simultaneously, using a dilution series of a specimen whose virus concentration was known.

The results obtained are shown in Table 2.

### Comparative Example 1

10 µL of a heparin solution (prepared by dissolving 160/USP units/mg of heparin in 5 mL of a 0.15 M sodium chloride solution) and 75 µL of 1 M MnCl₂ were added to 1 mL of human plasma containing 10⁴ copies/mL of HBV, followed by rotational stirring at room temperature for 20 minutes.

After the reaction was completed, the mixture was separated at 15,000 rpm for 10 minutes by means of a low-speed microcentrifuge, and the supernatant formed was discarded to obtain a sediment. To the sediment obtained in this way, 50 µL of saturated potassium bromide was added to solubilize the sediment (separated fraction). Its final volume was about 50 µL.

The separated fraction was taken to effect nucleic-acid extraction by a conventional method, and the quantity of DNA was determined by the TaqMan PCR process, using ABI PRISM TM7700 Sequence Detection System (made by Perkin Elmer Applied Biosystems Corp.). To make the determination of DNA, in the course of amplification made 40 times, the number of cycles (Th cycles) where fluorescence intensity exceeded a stated reference value (threshold: Th) was extrapolated on a calibration curve of DNA quantity Th cycles which was prepared by making measurement simultaneously, using a dilution series of a specimen whose virus concentration was known.

The results obtained are shown in Table 2.

**Table 2**

| Experiment No. | Type of particles | Amount of 1M MnCl₂ | Total collected DNA quantity (copies) | Collection rate (%) | Concentrating rate (times) |
|---|---|---|---|---|---|
| 1 | Example 1 * | 0 | 6.78 X 10⁶ | 67.8 | 6.8 |
| 2 | Example 2 * | 0 | 5.90 X 10⁶ | 59.0 | 5.9 |
| 3 | Example 3 * | 0 | 8.30 X 10⁶ | 83.0 | 8.3 |
| 4 | Example 4 | 0 | 1.06 X 10⁷ | 100 | 10 |
| 5 | Example 5 * | 0 | 1.03 X 10⁷ | 100 | 10 |
| 6 | Example 6 | 0 | 1.10 X 10⁷ | 100 | 10 |
| 7 | Example 2 * | 100 | 9.82 X 10⁶ | 98.2 | 9.8 |
| 8 | Comparative Example 1 | 75 | - | 0 | - |

| | | | | | |
|---|---|---|---|---|---|
| *Reference Example | | | | | |

As described above, the use of the virus-binding particles of the present invention enables simultaneous treatment of a large number of specimens with ease to separate and concentrate viruses by a simple means of making centrifugation or making magnetism act. Besides, the resultant samples containing separated concentrated viruses do not adversely affect the treatment for nucleic-acid amplification. The method of separating viruses by using such particles enables viruses to be separated in a good efficiency and in a short time, from specimens containing viruses in a very small quantity. Also, the method of detecting viruses enables detection of viruses at a high precision.

The virus-binding particles used in the present invention, having adsorbed viruses thereon from specimens, can also be used for immunological assay.

## Claims

1. A method of separating viruses, comprising the steps of:
adding virus-binding particles to a sample which possibly contains viruses, to allow the viruses to bind to the particles; said virus-binding particles having a particle diameter of 0.05 µm to 300 µm and having a polyalkylimine at their surfaces; and
separating from the sample the particles to which the viruses have bound to collect the particles.

2. The method of separating viruses according to claim 1, wherein a polyvalent metal compound is added to said sample together with said particles.

3. The method of separating viruses according to claim 1 or 2, wherein said polyalkylimine is polyethyleneimine.

4. A method of detecting viruses, comprising the steps of:
adding virus-binding particles to a sample which possibly contains viruses, to allow the viruses to bind to the particles; said virus-binding particles having a particle diameter of 0.05 µm to 300 µm and having a polyalkylimine at their surfaces;
separating viruses from the sample by separating from the sample the particles to which the viruses have bound, to collect the viruses; and
subjecting the viruses thus separated, to a nucleic-acid amplification test.

5. The method of detecting viruses according to claim 4, wherein a polyvalent metal compound is added to said sample together with said particles.

6. The method of detecting viruses according to claim 4 or 5, wherein said polyalkylimine is polyethyleneimine.

7. A method of detecting viruses, comprising the steps of:
adding virus-binding particles to a sample which possibly contains viruses, to allow the viruses to bind to the particles; said virus-binding particles having a particle diameter of 0.05 µm to 300 µm and having a polyalkylimine at their surfaces;
separating viruses from the sample by separating from the sample the particles to which the viruses have bound; and
subjecting the viruses bound to the particles, to an immunological assay.

8. The method of detecting viruses according to claim 7, wherein a polyvalent metal compound is added to said sample together with said particles.

9. The method of detecting viruses according to claim 7 or 8, wherein said polyalkylimine is polyethyleneimine.

## Patentansprüche

1. Verfahren zur Abtrennung von Viren, das die nachstehenden Schritte umfasst:
die Zugabe von viren-bindenden Teilchen zu einer Probe, die möglicherweise Viren enthält, um eine Bindung der Viren an die Teilchen zu ermöglichen; wobei die viren-bindenden Teilchen einen Teilchendurchmesser von 0,05 µm bis 300 µm aufweisen und ein Polyalkylimin auf ihren Oberflächen tragen; und
das Abtrennen der Teilchen, an die die Viren gebunden wurden, von der Probe, um die Teilchen zu gewinnen.

2. Verfahren zur Abtrennung von Viren nach Anspruch 1, in dem eine mehrwertige Metallverbindung zusammen mit den Teilchen zu der Probe gegeben wird.

3. Verfahren zur Abtrennung von Viren nach Anspruch 1 oder Anspruch 2, in dem es sich bei dem Polyalkylimin um Polyethylenimin handelt.

4. Verfahren zum Nachweis von Viren, das die nachstehenden Schritte umfasst:
die Zugabe von viren-bindenden Teilchen zu einer Probe, die möglicherweise Viren enthält, um eine Bindung der Viren an die Teilchen zu ermöglichen; wobei die viren-bindenden Teilchen einen Teilchendurchmesser von 0,05 µm bis 300 µm aufweisen und ein Polyalkylimin auf ihren Oberflächen tragen;
das Abtrennen der Viren von der Probe durch Abtrennen der Teilchen, an die die Viren gebunden wurden, von der Probe, um die Viren zu gewinnen; und
das Unterziehen der so abgetrennten Viren einem Nucleinsäure-Amplifikationstest.

5. Verfahren zum Nachweis von Viren nach Anspruch 4, in dem eine mehrwertige Metallverbindung zusammen mit den Teilchen zu der Probe gegeben wird.

6. Verfahren zum Nachweis von Viren nach Anspruch 4 oder Anspruch 5, in dem es sich bei dem Polyalkylimin um Polyethylenimin handelt.

7. Verfahren zum Nachweis von Viren, das die nachstehenden Schritte umfasst:
die Zugabe von viren-bindenden Teilchen zu einer Probe, die möglicherweise Viren enthält, um eine Bindung der Viren an die Teilchen zu ermöglichen; wobei die viren-bindenden Teilchen einen Teilchendurchmesser von 0,05 µm bis 300 µm aufweisen und ein Polyalkylimin auf ihren Oberflächen tragen;
das Abtrennen der Viren von der Probe durch Abtrennen der Teilchen, an die die Viren gebunden wurden, von der Probe; und
das Unterziehen der an die Teilchen gebundenen Viren einem Immunassay.

8. Verfahren zum Nachweis von Viren nach Anspruch 7, in dem eine mehrwertige Metallverbindung zusammen mit den Teilchen zu der Probe gegeben wird.

9. Verfahren zum Nachweis von Viren nach Anspruch 7 oder Anspruch 8, in dem es sich bei dem Polyalkylimin um Polyethylenimin handelt.

## Revendications

1. Procédé de séparation des virus, comprenant les étapes de :
addition de particules se liant aux virus à un échantillon qui contient éventuellement des virus, pour permettre aux virus de se lier aux particules ; lesdites particules se liant aux virus ayant un diamètre de particule de 0,05 µm à 300 µm et ayant une polyalkylimine à leur surface ; et
séparation à partir de l'échantillon des particules auxquelles les virus se sont liés pour recueillir les particules.

2. Procédé de séparation des virus selon la revendication 1, dans lequel un composé métallique polyvalent est ajouté au dit échantillon en même temps que lesdites particules.

3. Procédé de séparation des virus selon la revendication 1 ou 2, dans lequel ladite polyalkylimine est la polyéthylèneimine.

4. Procédé de détection des virus, comprenant les étapes de :
addition de particules se liant aux virus à un échantillon qui contient éventuellement des virus, pour permettre aux virus de se lier aux particules ; lesdites particules se liant aux virus ayant un diamètre de particule de 0,05 µm à 300 µm et ayant une polyalkylimine à leur surface ;
séparation des virus de l'échantillon en séparant de l'échantillon les particules auxquelles les virus se sont liés, pour recueillir les virus ; et
soumission des virus ainsi séparés, à un test d'amplification d'acide nucléique.

5. Procédé de détection des virus selon la revendication 4, dans lequel un composé métallique polyvalent est ajouté au dit échantillon en même temps que lesdites particules.

6. Procédé de détection des virus selon la revendication 4 ou 5, dans lequel ladite polyalkylimine est la polyéthylèneimine.

7. Procédé de détection des virus, comprenant les étapes de :
addition de particules se liant aux virus à un échantillon qui contient éventuellement des virus, pour permettre aux virus de se lier aux particules ; lesdites particules se liant aux virus ayant un diamètre de particule de 0,05 µm à 300 µm et ayant une polyalkylimine à leur surface.
séparation des virus de l'échantillon en séparant de l'échantillon les particules auxquelles les virus se sont liés ; et
soumission des virus liés aux particules, à un essai immunologique.

8. Procédé de détection des virus selon la revendication 7, dans lequel un composé métallique polyvalent est ajouté au dit échantillon en même temps que lesdites particules.

9. Procédé de détection des virus selon la revendication 7 ou 8, dans lequel ladite polyalkylimine est la polyéthylèneimine.
